# FASCICULE DE BREVET EUROPEEN

(11) **EP 0 782 847 B1**
(45) Date de publication et mention de la délivrance du brevet: **26.08.1998**
(21) Numéro de dépôt: 96402685.0
(22) Date de dépôt: 10.12.1996
(51) Int. Cl.: A61K 7/00

(54) **Compositions aqueuses contenant des vésicules lipidiques non-ioniques et au moins un pigment non-enrobé dispersé dans la phase aqueuse, procédé de préparation, utilisations**
Wässrige Zusammensetzung, die nichtionische Lipidvesikel und mindestens ein nicht umhülltes, in der wässrigen Phase dispergiertes Pigment enthalten, ihr Herstellungsverfahren und Verwendungen
Aqueous composition containing lipide nonionic vesicles and at least a non coated pigment dispersed in the aqueous phase, process for this preparation, uses

(30) Priorité: 03.01.1996 FR 9600030
(43) Date de publication de la demande: 09.07.1997
(73) Titulaire: L'OREAL, 75008 Paris (FR)
(72) Inventeur: Terren, Nadia, 94550 Chevilly Larue (FR); Bouchard, Fabienne, 94800 Villejuif (FR); Michelet, Jacques, 91160 Champlan (FR)
(74) Mandataire: Miszputen, Laurent

(56) Documents cités:
- EP-A- 0 485 251
- EP-A- 0 509 338
- EP-A- 0 523 418
- EP-A- 0 537 092
- EP-A- 0 582 503
- WO-A-93/15708
- FR-A- 2 315 991
- FR-A- 2 681 329
- GB-A- 2 136 762
- SOAP,COSMETICS,CHEMICAL SPECIALTIES, vol. 69, no. 5, Mai 1993, NEW YORK, pages 37-40, XP000369584 KENNETH ABATE,PH.D.:

## Description

La présente invention concerne des compositions comprenant une dispersion aqueuse contenant au moins un pigment non-enrobé dispersé dans la phase aqueuse et des vésicules lipidiques à coeur aqueux à base d'ester d'acide gras, leur procédé de préparation et leurs utilisations en cosmétique ou dermatologie.

On sait que certains lipides amphiphiles possèdent la propriété de former des phases mésomorphes, dont l'état d'organisation est intermédiaire entre l'état cristallin et l'état liquide et que, parmi eux, certains sont susceptibles de gonfler en présence d'une solution aqueuse pour former une phase lamellaire puis, après agitation, former des vésicules ou sphérules à coeur aqueux dispersées dans une phase aqueuse. Ces vésicules sont formées par une membrane constituée par des feuillets sensiblement concentriques comportant une ou plusieurs couches multimoléculaires, de préférence bimoléculaires, encapsulant une phase aqueuse.

Les vésicules à coeur aqueux (encapsulant une phase aqueuse) susmentionnées peuvent être préparées par de nombreux procédés. Selon un premier procédé, qui est par exemple décrit par BANGHAM et al. (J. Mol. Bio., 13, 1965 - pages 238 à 262), on dissout la phase lipidique sur les parois d'un flacon par évaporation du solvant, on introduit la phase à encapsuler sur le film lipidique et on agite le mélange mécaniquement jusqu'à obtention de la dispersion de vésicules à la taille désirée ; on obtient ainsi une dispersion de vésicules encapsulant une phase aqueuse, la phase aqueuse encapsulée et la phase aqueuse de dispersion étant identiques. Selon un second procédé dit "par cofusion des lipides", décrit par exemple dans FR-A-2315991, on prépare la phase lipidique par mélange du (des) lipide(s) amphiphile(s) et des éventuels additifs, à une température où le mélange est fondu, si le mélange n'est pas liquide à température ambiante ; on forme une phase lamellaire, par introduction de la phase aqueuse à encapsuler ; puis on disperse la phase lamellaire sous forme de vésicules, à l'aide d'un ultra-disperseur, d'un homogénéiseur ou d'ultrasons, dans une phase aqueuse de dispersion. Dans une variante de ce procédé, la formation de la phase lamellaire ne constitue pas un stade séparé du procédé. Les vésicules obtenues par ces deux procédés sont généralement de type "multifeuillet". Pour obtenir des vésicules de type "monofeuillet", on peut utiliser l'enseignement de FR-A-2543018.

Quel que soit le procédé utilisé, les vésicules à coeur aqueux sont obtenues sous forme de dispersion dans une phase aqueuse.

De façon connue, les vésicules de lipides amphiphiles à coeur aqueux peuvent contenir des actifs cosmétiques ou pharmaceutiques soit dans la phase aqueuse encapsulée si lesdits actifs sont hydrosolubles, soit dans la membrane lipidique s'ils sont oléosolubles. Des actifs peuvent également être présents dans la phase aqueuse de dispersion.

Les lipides amphiphiles utilisés pour l'obtention des vésicules sont des lipides ayant pour formule générale :

X - Y

formule dans laquelle X représente un groupe hydrophile et Y représente un groupe lipophile. Les lipides amphiphiles peuvent être des lipides ioniques, pour lesquels le groupe X est ionique, ou des lipides non-ioniques pour lesquelles le groupe X est non-ionique.

De façon connue, on peut utiliser pour la fabrication des vésicules à coeur aqueux, des mélanges de lipides amphiphiles ioniques, des mélanges de lipides amphiphiles non-ioniques et des mélanges de ces deux types de lipides.

On a proposé dans la demande FR-A-2694884 des dispersions de vésicules lipidiques à coeur aqueux non-ioniques constitués d'esters de polyol et d'acide gras en C₅-C₁₇.

On a proposé également dans la demande de brevet non publiée n° 95-02199 des dispersions de vésicules lipidiques à coeur aqueux, non-ioniques, à base d'estersd'acidegras d'α-butylglucoside.

Ces vésicules lipidiques à coeur aqueux à base d'esters d'acide gras sont particulièrement intéressants en cosmétique et en dermatologie, dans la mesure où ils présentent un bon taux d'encapsulation d'actifs hydrosolubles ou liposolubles, une bonne dégradabilité sous l'action du pH de la peau ou par les enzymes de la peau, une bonne stabilité dans l'eau.

Ils sont particulièrement adaptés pour la réalisation d'émulsions huile-dans-eau telles que des crèmes, des laits, des lotions ou des sérums pour le soin de la peau et/ou du cuir chevelu, en tant qu'agents dispersants de la phase huileuse dans la phase aqueuse.

Pour la fabrication de fonds de teint, de crèmes teintées, de correcteurs ou d'embellisseurs de teint, on utilise généralement des pigments tels que les oxydes de fer ou les oxydes de titane pour apporter l'effet couvrant et/ou l'effet couleur sur le visage.

Un objectif de la présente invention est de pouvoir réaliser des compositions sous forme de dispersion huile-dans-eau de vésicules lipidiques à coeur aqueux à base d'esters d'acide gras et contenant dans la phase aqueuse de la dispersion des pigments non-enrobés.

Jusqu'à présent, les compositions contenant une dispersion de vésicules lipidiques à coeur aqueux de ce type ne pouvaient contenir dans la phase aqueuse de la dispersion que des pigments enrobés par des silicones, des composés fluorés ou des acides aminés. De telles compositions sont décrites dans la demande EP-A-582503.

En effet, la dispersion des pigments non-enrobés dans la phase aqueuse dans laquelle sont également dispersés les vésicules lipidiques à coeur aqueux à base d'esters d'acide gras, déstabilise l'émulsion. Leur utilisation conduit à la formation d'amas de pigment qui ruinent les qualités cosmétiques et esthétiques du produit final : couleur hétérogène, séparation de phases voire irréalisation de la dispersion.

L'utilisation des pigments enrobés dans les dispersions huile-dans-eau de vésicules lipidiques à coeur aqueux constituées d'ester d'acide gras pose également un certain nombre de problèmes à la fabrication. La mouillabilité moindre des pigments enrobés dans la phase grasse et dans la phase aqueuse nuit à l'homogénéité et à la stabilité de l'émulsion. Leur dispersion nécessite une agitation plus poussée et plus longue au moyen d'un homogénéisateur classique du type turbine.

La demanderesse a découvert de manière surprenante que l'on pouvait incorporer dans la phase aqueuse d'une dispersion de vésicules lipidiques à coeur aqueux constituées d'esters d'acide gras, des pigments non-enrobés sans les inconvénients énumérés ci-dessus, en utilisant un dispositif d'homogénéisation à haute pression. Les dispersions huile-dans-eau ainsi obtenues sont stables, présentent une couleur homogène et ne contiennent pas ou pratiquement pas d'amas de pigment.

Un objet de l'invention consiste donc en de nouvelles compositions sous forme de dispersion huile-dans-eau contenant au moins dans la phase aqueuse un pigment non-enrobé et des vésicules lipidiques à coeur aqueux dont la membrane est formée à partir d'au moins un ester d'acide gras.

Un autre objet de l'invention consiste en un procédé de dispersion d'une charge dans une dispersion huile-dans-eau de vésicules lipidiques, par homogénéisation haute pression en particulier d'un pigment non-enrobé dans une dispersion huile-dans-eau de vésicules lipidiques à coeur aqueux dont la membrane est formée à partir d'au moins un ester d'acide gras.

Un autre objet consiste en un procédé de préparation des compositions de l'invention.

Les compositions selon l'invention sont essentiellement caractérisées par le fait qu'elles contiennent au moins une dispersion huile-dans-eau comprenant des vésicules lipidiques à coeur aqueux dont la membrane lipidique est formée à partir d'au moins un ester d'acide gras et au moins un pigment non-enrobé dispersé dans la phase aqueuse de dispersion.

Les compositions selon l'invention sont plus particulièrement des dispersions huile-dans-eau dans lesquelles les vésicules à coeur aqueux agissent comme agent dispersant des goutelettes d'huile dans la phase aqueuse de la dispersion.

On entend, par pigment, toute charge pulvérulente, minérale ou organique, insoluble dans une dispersion huile-dans-eau et présentant des propriétés colorantes et/ou couvrantes sur les matières kératiniques en particulier la peau.

Les esters d'acide gras constituant la membrane des vésicules lipidiques à coeur aqueux de l'invention sont choisis, de préférence, dans le groupe formé par:
(i) les esters de polyol et d'acide gras, polyoxyéthylénés ou non ;
(ii) les esters d'acide gras d'α-butylglycoside.

Les esters de polyol et d'acide gras sont de préférence choisis parmi les mélanges d'esters d'au moins un polyol choisi dans le groupe formé par le polyéthylèneglycol comportant de 1 à 60 unités oxyde d'éthylène, le sorbitane, le sorbitane portant 2 à 60 unités d'oxyde d'éthylène, le glycérol portant 2 à 30 unités d'oxyde d'éthylène, les polyglycérols comportant 2 à 15 unités glycérol, les sucroses, les glucoses portant 2 à 30 unités oxyde d'éthylène, et au moins un acide gras comportant une chaîne alkyle en C₅-C₁₇, saturée ou non saturée, linéaire ou ramifiée, le nombre de chaînes alkyle par groupe polyol étant compris entre 1 et 10.

Les esters de polyol et d'acides gras en C₅-C₁₇ particulièrement préférés sont ceux répondant à la formule : 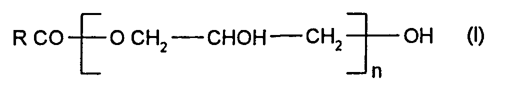 où n est une valeur statistique et qui peut contenir des proportions diverses d'esters pour lesquels n = 1, n = 2, n = 3, n = 4, etc ... ; c'est aussi le cas des esters comportant plusieurs chaînes alkyle dans leur partie lipophile, tels que les cocoates, qui contiennent des chaînes alkyles en C₅-C₁₇ ou les isostéarates où les chaînes alkyle sont en C₁₇ sont un mélange complexe de formes isomères ; c'est également le cas des produits constitués par des mélanges de mono-, di-, tri- ou polyesters d'un même polyol.

Parmi les produits commerciaux utilisables selon l'invention et ayant la structure d'un mélange d'esters de polyol et d'acide gras en C₅-C₁₇ tel que défini ci-dessus, on peut citer :
- les esters partiels de sorbitane (ou anhydride de sorbitol) et d'acide gras, vendus sous les dénominations commerciales "SPAN 20, 40, 60 et 80" par la Société "ICI";
- l'isostéarate de sorbitane, vendu sous la dénomination commerciale "Sl 10 R NIKKOL" par la Société "NIKKO" ;
- le stéarate de sorbitane portant 4 unités oxyde d'éthylène, vendu sous la dénomination "TWEEN 61" par la Société "ICI" ;
- le stéarate de polyéthylèneglycol à 8 unités oxyde d'éthylène vendu sous le nom 〈〈MYR J 45〉〉 par ICI ;
- le monostéarate du polyéthylène glycol de formule

   OHCH₂(CH₂OCH₂)ₙCH₂OH

   formule dans laquelle n est égal à 4, vendu sous la dénomination "MYS 4" par la Société "NIKKO" ;
- le stéarate de polyéthylèneglycol de poids moléculaire 400, qualité chimique ou qualité produite par biotechnologie, vendu par la Société "UNICHEMA" ;
- le stéarate de diglycéryle portant 4 unités d'oxyde d'éthylène, vendu sous la dénomination "HOSTACERINE DGS" par la Société "HOESCHT" ;
- le stéarate de tétraglycérol, vendu sous la dénomination "TETRAGLYN 1S" par la Société "NIKKO" ;
- l'isostéarate de diglycéryle, vendu par la Société "SOLVAY" ;
- le distéarate de diglycéryle, vendu sous la dénomination "EMALEX DSG 2" par la Société "NIHON" ;
- les mono-, di- et tri-palmitostéarate de sucrose, vendu sous les dénominations "F50, F70, F110 et F160 CRODESTA" par la Société "CRODA" ;
- le mélange de mono- et di-palmitostéarate de sucrose, vendu sous la dénomination "GRILLOTEN PSE 141 G" par la Société "GRILLO" ;
- le mélange de stéarate de sucrose et de cocoate de sucrose, vendu sous la dénomination "ARLATONE 2121" par la Société "ICI" ;
- le distéarate de méthylglucose portant 20 unités oxyde d'éthylène, vendu sous la dénomination "GLUCAM E20 DISTEARATE" par la Société "AMERCHOL".

Les esters d'acide gras d'α-butylglucoside utilisés selon l'invention sont de préférence, soit des mélanges d'esters de différents acides gras d'α-butylglucoside dont les différentes chaînes grasses comportent, l'une par rapport à l'autre, un nombre d'atomes de carbone voisin (par exemple différent de 1 ou 2) soit des mélanges de mono-, di- tri- ou polyesters d'un même acide gras d'α-butylglucoside.

Le (ou les) ester(s) d'acide gras d'α-butylglucoside utilisé(s) selon l'invention comporte(nt) de préférence une chaîne grasse ayant de 8 à 24 atomes de carbone, plus préférentiellement de 12 à 22 atomes de carbone et plus particulièrement de 14 à 18 atomes de carbone.

On peut citer par exemple, les esters d'acide laurique (C₁₂), myristique (C₁₄), palmitique (C₁₆), stéarique (C₁₈), béhénique (C₂₂) d'α-butylglucoside.

On utilise plus particulièrement un mélange de mono- et de diester d'acide palmitique d'α-butylglucoside obtenu selon le procédé de fabrication enzymatique décrit dans les vésicules lipidiques conformes à l'invention.

Les esters d'acide gras d'α-butylglucoside conformes à l'invention peuvent être préparés à partir d'α-butylglucoside obtenu selon le procédé de fabrication enzymatique décrit dans la demande de brevet FR-A-2680373 qui consiste à mettre en contact le butanol avec de l'amidon, des maltodextrines ou du maltose en présence d'une préparation enzymatique purifiée présentant une activité d'a-transglucosylation. Les esters d'acide gras d'α-butylglucoside peuvent être synthétisés en faisant réagir l'acide gras ou le mélange d'acide gras correspondants avec l'α-butylglucoside selon des procédés classiques d'estérification comme ceux utilisant une lipase ou un équivalent.

Les vésicules lipidiques à coeur aqueux conformes à l'invention, de préférence, comprennent une membrane de phase lipidique formée à partir d'au moins un ester d'acide gras tel que défini ci-dessus et d'au moins un lipide amphiphile ionique.

Les lipides amphiphiles ioniques asociés à l'ester ou au mélange d'esters d'acide gras, utilisés pour la fabrication des vésicules lipidiques de l'invention, sont choisis de préférence dans le groupe formé par :
1) les lipides anioniques neutralisés, ces lipides anioniques étant de préférence, choisis parmi :
   - les sels alcalins du dicétylphosphate, et du dimyristylphosphate en particulier les sels de Na et K ;
   - les sels alcalins du cholestérol-sulfate, en particulier le sel de Na ;
   - les sels de lipoaminoacides tels que les acylglutamates mono- et disodique ;
   - le sel de sodium de l'acide phosphatidique ;
2) les lipides amphotères, ces lipides amphotères étant, de préférence, des phospholipides, en particulier la phosphatidyléthanolamine de soja pure ;
3) les dérivés alkylsulfoniques, ces dérivés étant de préférence, les composés de formule : 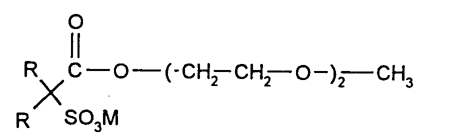 formule dans laquelle R représente des radicaux en C₁₂-C₂₂, en particulier les radicaux C₁₆H₃₃ et C₁₈H₃₇, pris en mélange ou séparément et M est un métal alcalin, de préférence le sodium.

De façon préférentielle, le rapport en poids entre la quantité d'ester d'acide gras et la quantité de lipide amphiphile ionique est compris entre 50/1 et 50/25 et le rapport en poids entre la quantité de phase lipidique et la quantité de phase aqueuse est comprise entre 1/1000 et 300/1000.

Les vésicules lipidiques à coeur aqueux peuvent être préparés par tout procédé connu de fabrication des vésicules lipidiques amphiphiles et plus particulièrement par le procédé dit "par cofusion des lipides".

On peut, de façon connue, incorporer dans la phase lipidique constituant la membrane lipidique des vésicules, au moins un additif qui a pour fonction principale de diminuer la perméabilité des vésicules, de prévenir leur floculation et leur fusion et d'augmenter le taux d'encapsulation.

Selon l'invention, on peut ajouter à la phase lipidique au moins un additif choisi, de préférence, dans le groupe formé par:
- les stérols et notamment les phytostérols et le cholestérol,
- les alcools et diols à longue chaîne,
- les amines à longue chaîne et leurs dérivés ammonium quaternaire.

Ces additifs peuvent éventuellement avoir une activité cosmétique et/ou dermopharmaceutique. C'est, par exemple, le cas du cholestérol.

Les vésicules des compositions selon l'invention peuvent contenir, de façon connue, un ou plusieurs composé(s) actif(s) ayant une activité cosmétique et/ou dermopharmaceutique, qui, selon leurs caractéristiques de solubilité, peuvent avoir différentes localisations. Si les actifs sont hydrosolubles, on les introduit dans la phase aqueuse encapsulée des vésicules. Si les actifs sont liposolubles, on les introduit dans la phase lipidique constituant la membrane. Si les actifs sont amphiphiles, ils se répartissent entre la phase lipidique et la phase aqueuse encapsulée avec un coefficient de partage, qui varie selon la nature de l'actif amphiphile et les compositions respectives de la phase lipidique et de la phase aqueuse encapsulée.

Les actifs hydrosolubles sont, par exemple, la glycérine, le sorbitol, l'érythrulose et les antibiotiques. Les actifs liposolubles ou partiellement liposolubles (amphiphiles) sont choisis parmi ceux qui n'augmentent pas de façon significative la perméabilité des vésicules, ne provoquent pas leur floculation et leur fusion et ne diminuent pas le taux d'encapsulation. On utilise avantageusement des actifs liposolubles qui constituent également des additifs.

Les actifs liposolubles préférés selon l'invention sont choisis dans le groupe formé par:
- les sphingomyélines,
- les glycocéramides, en particulier ceux issus de germe de blé et
- les céramides naturels ou de synthèse, de préférence ceux décrits dans la demande de brevet français n° 9102091 déposée le 21 février 1991, ayant pour formule : 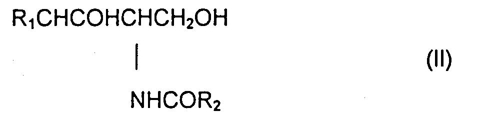 formule dans laquelle :
   R₁ représente un radical alkyle ou alcényle en C₁₁-C₂₁,
   R₂ représente :
      - un radical hydrocarboné en C₁₁-C₂₃ saturé,
      - un mélange de radicaux hydrocarbonés en C₁₁-C₁₉, linéaires, saturés et portant au moins une insaturation éthylénique, de préférence 1 ou 2, dans lequel la proportion de radicaux saturés ne peut excéder 35 %,
      le céramide de formule (II) étant sous forme d'un mélange racémique de diastéréoisomères érythro et thréo dans des proportions érythro/thréo comprises entre 85/15 et 60/40.

Selon l'invention, on introduit, de préférence, dans la phase lipidique constituant la membrane un mélange de céramide(s) et de cholestérol. En effet, l'utilisation de ce mélange est particulièrement intéressante, car elle permet de reconstituer les lipides de la peau, lors de la dégradation des vésicules de la dispersion selon l'invention, sur la peau.

La dispersion conforme à l'invention contient de préférence une huile dispersée dans la phase aqueuse par les vésicules lipidiques à coeur aqueux.

L'huile peut notamment être choisi dans le groupe formé par :
- les huiles animales ou végétales formées par des esters d'acide gras et de polyols, en particulier les triglycérides liquides, par exemple les huiles de tournesol, de maïs, de soja, de courge, de pépins de raisin, de jojoba, de sésame, de noisette, les huiles de poisson, le tri-caprocaprylate de glycérol, ou les huiles végétales ou animales de formule R₉COOR₁₀, formule dans laquelle R₉ représente le reste d'un acide gras supérieur comportant de 7 à 19 atomes de carbone et R₁₀ représente une chaîne hydrocarbonée contenant de 3 à 20 atomes de carbone, par exemple l'huile de Purcellin ;
- des huiles essentielles naturelles ou synthétiques telles que, par exemple, les huiles d'eucalyptus, de lavandin, de lavande, de vétiver, de litsea cubeba, de citron, de santal, de romarin, de camomille, de sariette, de noix de muscade, de cannelle, d'hysope, de carvi, d'orange, de géraniol, de cade et de bergamote ;
- des hydrocarbures, tels que l'hexadécane et l'huile de paraffine ;
- des carbures halogénés, notamment des fluorocarbures tels que des fluoroamines par exemple la perfluorotributylamine, des hydrocarbures fluorés, par exemple le perfluorodécahydronaphtalène, des fluoroesters et des fluoroéthers ;
- des silicones, par exemple les polysiloxanes, les polydiméthylsiloxanes et les fluorosilicones ;
- des esters d'acide minéral et d'un alcool ; et
- des éthers et des polyéthers.

La phase aqueuse de dispersion peut aussi également contenir des actifs cosmétiques et/ou dermopharmaceutiques, hydrosolubles. L'huile peut éventuellement contenir un actif liposoluble.

Les compositions selon l'invention contiennent des pigments minéraux et/ou des pigments organiques non-enrobés.

Parmi les pigments organiques, on peut citer les laques telles que les laques de calcium de D et C Red n° 7, les laques de baryum des D et C Red n° 6 et 9, les laques d'aluminium des D et C Red n° 3 et de D et C Yellow n° 5 et les laques de zirconium de D et C Orange n° 5. Parmi les pigments minéraux, on peut citer : les oxydes de fer (rouge, brun, noir et jaune), les oxydes de chrome, les outre-mers (polysulfures d'aluminosilicates), le pyrophosphate de manganèse et le bleu de prusse (ferrocyanure ferrique), les oxydes de titane.

Les pigments non-enrobés sont présents dans les compositions de l'invention dans des proportions allant de préférence, de 1 à 20 % en poids et plus préférentiellement de 2 à 12 % en poids par rapport au poids total de la composition.

Les compositions selon l'invention peuvent contenir également dans la phase aqueuse des agents matifiants tels que par exemple le carbonate de magnésium, de l'amidon ou des amidons modifiés, de la poudre de polyéthylène, de la poudre de zinc, de l'oxyde de zinc, du stéarate de magnésium ou de zinc, des microbilles de résine siliconée telles que le produit vendu sous le nom TOSPEARL par la Société TOSHIBA, des microsphères de silice.

Les compositions selon l'invention peuvent également contenir des actifs cosmétiques ou dermatologiques tels que la vitamine A, la vitamine E, le palmitate de vitamine A et la vitamine F et des additifs tels que des conservateurs, des parfums, des filtres solaires, des anti-oxydants, des bactéricides, des hydratants, des charges telles que le talc, le kaolin, le mica ; des agents gélifiants, des colorants.

Dans la composition selon l'invention, les vésicules ont généralement un diamètre moyen compris entre 10 et 5 000 nm. Lorsque la phase aqueuse contient une dispersion de goutelettes d'huile, ces goutelettes ont de façon avantageuse un diamètre moyen compris entre 100 et 10 000 nm.

Un autre objet de l'invention est un procédé de dispersion d'une charge organique et/ou d'une charge minérale pulvérulente dans une dispersion huile-dans-eau de vésicules lipidiques, caractérisée par le fait que l'on mélange la dispersion de vésicules lipidiques et la ou les charges dans la phase aqueuse au moyen d'un dispositif d'homogénéisation à haute pression en un ou plusieurs passages.

Les dispositifs d'homogénéisation à haute pression utilisés selon l'invention sont choisis parmi notamment ceux du type RANNIE®, GAULIN®, ou SOAVIE®.

Les pressions utilisées sont de préférence comprises entre 400 et 800 bars (entre 4 et 8.10⁷ Pa) et plus particulièrement entre 450 et 600 bars (entre 4,5 et 6.10⁷ Pa). La température utilisée est de préférence la température ambiante.

Le procédé de dispersion conforme à l'invention permet, de manière inattendue, de disperser de façon fine et homogène des pigments non-enrobés à titre de charges dans des dispersions huile-dans-eau de vésicules lipidiques à coeur aqueux dont la membrane lipidique est formé à partir d'esters d'acide gras telles que définies ci-dessus.

Les compositions conformes à la présente invention telles que définies ci-dessus peuvent être préparées selon un procédé comprenant les étapes suivantes :
(a) on mélange une suspension aqueuse de pigments non-enrobés à une dispersion huileuse de vésicules lipidiques à coeur aqueux constitués d'esters d'acide gras, à température ambiante ;
(b) on homogénéise selon un procédé classique d'homogénéisation d'une dispersion huile-dans-eau (turbine) ;
(c) on effectue une homogénéisation haute pression selon le procédé de dispersion tel que décrit précédemment.

De façon préférentielle, on refroidit le mélange jusqu'à température ambiante, le mélange obtenu après chaque passage dudit mélange dans l'homogénéisateur haute pression par un courant d'air froid puis on désaère.

Un autre objet de l'invention consiste en des compositions à usage topique. Elles sont caractérisées par le fait qu'elles sont constituées par les compositions telles que définies ci-dessus.

Un autre objet de l'invention consiste en l'utilisation des compositions telles que définies précédemment comme base de produits pour le soin et/ou le maquillage du visage et/ou du corps et/ou du cuir chevelu.

Les produits obtenus à partir des compositions de l'invention peuvent être cosmétiques ou dermopharmaceutiques. Ils peuvent se présenter sous forme de dispersion plus ou moins épaissie, de gel, de crème, de lait ou de sérum. Parmi les compositions de maquillage, on peut citer les fonds de teint, les crèmes teintées, les correcteurs, les embellisseurs de teint.

L'invention porte également sur un procédé de traitement cosmétique de la peau et/ou du cuir chevelu, caractérisée par le fait qu'on applique sur la peau ou le cuir chevelu, une composition telle que définie précédemment.

Les exemples ci-après, donnés à titre purement illustratif et non limitatif permettront de mieux comprendre l'invention.

Dans tous les exemples donnés ci-après, les dispersions de vésicules sont préparées par le procédé dit "par cofusion des lipides" dans lequel :
- dans une première phase, on prépare la phase lipidique par mélange sous forme liquide de différents lipides amphiphiles la composant, et éventuellement associés à des additifs ou des actifs liposolubles, et on met la phase lipidique obtenue en présence d'une phase aqueuse contenant éventuellement des actifs hydrosolubles, de façon à obtenir une phase lamellaire puis on homogénéise l'ensemble ;
- et dans une deuxième phase, on soumet le mélange à une agitation énergique dans une homogénéiseur pour obtenir des vésicules dispersées dans une phase aqueuse de dispersion.

### Exemple 1 : Fond de teint

### Phase A₁

| | |
|---|---|
| - Palmitate de sorbitane commercialisé sous le nom SPAN 40 par la Société ICI | 2,85 g |
| - Cholestérol | 2,85 g |
| - Glutamate monosodique commercialisé sous le nom "ACYLGLUTAMATE HS11" par la Société AJINOMOTO | 0,3 g |
| - Tocophérol | 1,1 g |

### Phase A₂

| | |
|---|---|
| - Eau déminéralisée | 35 g |
| - Glycérine | 3 g |
| - Conservateur | 0,1 g |
| - Acide citrique anhydre | 0,02 g |

### Phase B₁

| | |
|---|---|
| - Phenyltriméthylsiloxysiloxane vendu sous le nom "DC556 FLUID COSMETIC" par DOW CORNING | 12 g |
| - Palmitate d'éthyl-2 hexyle vendu sous le nom "CERAPHIL 368" par la Société VAN DYK | 4 g |

### Phase B₂

| | |
|---|---|
| - Néopentanoate d'isostéaryle vendu sous le nom "CERAPHIL 375" par la Société VAN DYK | 4 g |
| - Conservateur | 0,15 g |

### Phase C₁

| | |
|---|---|
| - Silicate de magnésium et d'aluminium vendu sous le nom de "VEEGUM" par la Société VANDERBILT | 0,75 g |
| - Eau déminéralisée | 22,58 g |

### Phase C₂

| | |
|---|---|
| - Oxyde de fer jaune (pigment non-enrobé) | 0,98 g |
| - Oxydes de fer jaune et de fer brun (pigments non-enrobés) | 0,58 g |
| - Oxyde de fer noir (pigment non-enrobé) | 0,2 g |
| - Oxyde de titane (pigment non-enrobé) | 5,24 g |

### Phase D

| | |
|---|---|
| - Eau déminéralisée | 1 g |
| - Conservateur | 0,3 g |

### Phase E

| | |
|---|---|
| - Talc (charge) | 2 g |

### Phase F

| | |
|---|---|
| - Agent gélifiant | 1 g |

### MODE OPERATOIRE :

On fait fondre les vésicules lipidiques à 90° C (phase A₁). On hydrate avec la phase A₂ qui a été préparée à 85° C. On refroidit à 60° C. On passe deux fois à l'homogénéisateur haute pression à 500 bars et refroidit la dispersion de vésicules lipidiques obtenue.

On ajoute la phase grasse (B₁ + B₂) à ladite dispersion et on disperse les huiles dans la phase aqueuse avec l'homogénéisateur haute pressioon à 500 bars en 2 passages successifs.

On forme une suspension aqueuse de pigments non-enrobés en dispersant la phase C₂ dans la phase C₁ à 60° C. On refroidit à température ambiante. On aspire la dispersion de vésicules sur les pigments avec une pompe.

On passe le mélange à l'homogénéisateur haute pression en 2 passages successifs.

On refroidit et on désaére. On ajoute ensuite les phases D, E et F et on homogénéise le mélange final avec une turbine.

### Exemple 2 : Fond de teint

### Phase A₁

| | |
|---|---|
| - Palmitate de butylglucopyranoside | 2,7 g |
| - Cholestérol | 2,7 g |
| - Glutamate monosodique commercialisé sous le nom "ACYLGLUTAMATE HS11" par la Société AJINOMOTO | 0,6 g |

### Phase A₂

| | |
|---|---|
| - Eau déminéralisée | 40 g |
| - Glycérine | 2 g |
| - Conservateur | 0,1 g |

### Phase B₁

| | |
|---|---|
| - Huile de macadamia | 12 g |
| - Conservateur | 0,15 g |

### Phase B₂

| | |
|---|---|
| - Cyclopentadiméthylsiloxane (4cst) | 7,5 g |
| - Esters glycériques d'acides gras essentiels | 3,0 g |
| - Conservateur | 0,15 g |

### Phase C₁

| | |
|---|---|
| - Eau déminéralisée | 17,95 g |

### Phase C₂

| | |
|---|---|
| - Oxyde de fer jaune (pigment non-enrobé) | 0,69 g |
| - Oxydes de fer jaune et de fer brun (pigments non-enrobés) | 0,30 g |
| - Oxyde de fer noir (pigment non-enrobé) | 0,13 g |
| - Oxyde de titane (pigment non-enrobé) | 5,88 g |

### Phase D

| | |
|---|---|
| - Eau déminéralisée | 1 g |
| - Conservateur | 0,3 g |

### Phase E

| | |
|---|---|
| - Agent gélifiant | 3 g |

Le mode opératoire est identique à celui de l'exemple 1.

### Exemple 3 comparatif selon l'art antérieur : Fond de teint

### Phase A₁

| | |
|---|---|
| - Palmitate de sorbitane commercialisé sous le nom SPAN 40 par la Société ICI | 2,85 g |
| - Cholestérol | 2,85 g |
| - Glutamate monosodique commercialisé sous le nom "ACYLGLUTAMATE HS11" par la Société AJINOMOTO | 0,3 g |
| - Tocophérol | 1,1 g |

### Phase A₂

| | |
|---|---|
| - Eau déminéralisée | 35 g |
| - Glycérine | 3 g |
| - Conservateur | 0,1 g |
| - Acide citrique anhydre | 0,02 g |

### Phase B₁

| | |
|---|---|
| - Phenyltriméthylsiloxysiloxane vendu sous le nom "DC556 FLUID COSMETIC" par DOW CORNING | 12 g |
| - Palmitate d'éthyl-2 hexyle vendu sous le nom "CERAPHIL 368" par la Société VAN DYK | 4 g |

### Phase B₂

| | |
|---|---|
| - Néopentanoate d'isostéaryle vendu sous le nom "CERAPHIL 375" par la Société VAN DYK | 4 g |
| - Conservateur | 0,15 g |

### Phase C

| | |
|---|---|
| - Oxyde de fer jaune (pigment enrobé) | 0,98 g |
| - Oxydes de fer jaune et de fer brun (pigments enrobés) | 0,58 g |
| - Oxyde de fer noir (pigment enrobé) | 0,2 g |
| - Oxyde de titane (pigment enrobé) | 5,24 g |

### Phase D

| | |
|---|---|
| - Eau déminéralisée | 1 g |
| - Conservateur | 0,3 g |

### Phase E

| | |
|---|---|
| - Talc (charge) | 2 g |

### Phase F

| | |
|---|---|
| - Agent gélifiant | 1 g |

### MODE OPERATOIRE :

On fait fondre les vésicules lipidiques à 90° C (phase A₁). On hydrate avec la phase A₂ qui a été préparée à 85° C. On refroidit à 60° C. On passe deux fois à l'homogénéisateur haute pression à 500 bars et refroidit la dispersion de vésicules lipidiques obtenue.

On ajoute la phase grasse (B₁ + B₂) à ladite dispersion et on disperse les huiles dans la phase aqueuse avec l'homogénéisateur haute pression à 500 bars en 2 passages successifs.

On ajoute ensuite les phases C (pigments enrobés) , D, E et F et on homogénéise le mélange final avec une turbine.

### COMPARAISON ENTRE LA FORMULATION DE L'EXEMPLE 1 ET CELLE DE L'EXEMPLE COMPARATIF 2

On observe au microscope optique G x 100 la présence ou non d'amas de pigments et la qualité de l'émulsion.
- **EXEMPLE 1 (invention):**: Fine et homogène
Pas d'amas de pigments
- **EXEMPLE COMPARATIF 3 (art antérieur)** :: Moins fine et moins homogène
Amas de pigments de taille supérieure ou égale à 40 µm

## Revendications

1. Composition contenant une dispersion aqueuse de vésicules lipidiques à coeur aqueux, caractérisée par le fait que la dispersion comprend au moins un pigment non-enrobé dispersé dans la phase aqueuse et que les vésicules lipidiques à coeur aqueux ont une membrane formée à partir d'au moins un ester d'acide gras.

2. Composition selon la revendication 1, caractérisée par le fait qu'elle est une dispersion huile-dans-eau dans laquelle les vésicules lipidiques à coeur aqueux agissent comme agent dispersant des goutelettes d'huile dans la phase aqueuse de la dispersion.

3. Composition selon la revendication 1 ou 2, caractérisée par le fait que les esters d'acide gras constituant la membrane des vésicules lipidiques à coeur aqueux sont choisis dans le groupe formé par :
(i) les esters de polyol et d'acide gras, polyoxyéthylénés ou non ;
(ii) les esters d'acide gras d'α-butylglucoside.

4. Composition selon l'une quelconque des revendications 1 à 3, caractérisée par le fait que les esters de polyol et d'acides gras sont choisis parmi les mélanges d'au moins un polyol choisi dans le groupe formé par le polyéthylèneglycol comportant de 1 à 60 unités d'oxyde d'éthylène, le sorbitane, le sorbitane portant 2 à 60 unités d'oxyde d'éthylène, le glycérol portant 2 à 30 unités d'oxyde d'éthylène, les polyglycérols comportant 2 à 15 unités glycérol, les sucroses, les glucoses portant 2 à 30 unités d'oxyde d'éthyléne et d'au moins un acide gras comportant une chaîne alkyle en C₅-C₁₇ saturée ou non-saturée, linéaire ou ramifiée ; le nombre de chaînes alkyle par groupe polyol étant compris entre 1 et 10.

5. Composition selon la revendication 3, caractérisée par le fait que les esters d'acide gras d'α-butylglucoside comportent une chaîne grasse de 8 à 24 atomes de carbone.

6. Composition selon la revendicationi 5, caractérisée par le fait que les esters d'acide gras, d'α-butylglucoside sont des mélanges d'esters de différents acides gras d'-α-butylglucoside dont les différentes chaînes comportent l'une par rapport à l'autre un nombre d'atomes de carbone voisin ou bien des mélanges de mono-, di-, tri- ou polyesters d'un même acide gras d'α-butylglucoside.

7. Composition selon la revendication 5 ou 6, caractérisée par le fait que l'ester d'acide gras d'α-butylglucoside est un mélange de mono- et de diester d'acide palmitique d'α-butylglucoside.

8. Composition selon l'une quelconque des revendications 1 à 7, caractérisée par le fait que les vésicules lipidiques à coeur aqueux sont constituées d'une membrane formée à partir d'au moins un ester d'acide gras et d'au moins un lipide amphiphile ionique.

9. Composition selon la revendication 8, caractérisée par le fait que les lipides amphiphiles ioniques sont choisis parmi les lipides anioniques neutralisés, les lipides amphotères ou les dérivés alkylsulfoniques.

10. Composition selon la revendication 9, caractérisée par le fait que les lipides amphiphiles ioniques sont choisis dans le groupe formé par :
- les sels alcalins de dicétylphosphate et du dimyristylphosphate ;
- les sels alcalins du cholestérol sulfate ;
- les sels alcalins de lipoaminoacides ;
- le sel de sodium de l'acide phosphatidique ;
- les phospholipides ;
- les dérivés alkylsulfoniques de formule : 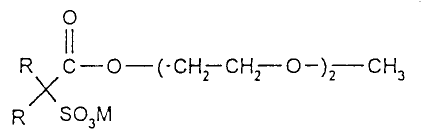 où R représente des radicaux en C₁₂-C₂₂, en particulier les radicaux C₁₆H₃₃ et C₁₈H₃₇, pris en mélange ou séparément et M est un métal alcalin, de préférence le sodium.

11. Composition selon l'une quelconque des revendications 8 à 10, caractérisée par le fait que le rapport en poids de la quantité d'ester d'acide gras sur la quantité de lipide amphiphile ionique est compris entre 50/1 et 50/25 et que le rapport en poids de la quantité de phase lipidique sur la quantité de phase aqueuse est comprise entre 1/1000 et 300/1000.

12. Composition selon l'une quelconque des revendications 1 à 11, caractérisée par le fait que la membrane lipidique des vésicules à coeur aqueux contient au moins un additif.

13. Composition selon la revendication 12, caractérisée par le fait que l'additif est choisi dans le groupe formé par les stérols, les alcools et diols à longue chaîne, les amines à longue chaîne et leurs dérivés quaternaires.

14. Composition selon l'une quelconque des revendications 1 à 13, caractérisée par le fait que les vésicules lipidiques à coeur aqueux contiennent au moins un actif cosmétique et/ou dermopharmaceutique.

15. Composition selon la revendication 13, caractérisée par le fait que les vésicules lipidiques à coeur aqueux encapsulent au moins un actif hydrosoluble et/ou contiennent dans la membrane lipidique au moins un actif lipophile et /ou contiennent au moins un actif amphiphile qui se répartit entre la membrane et la phase aqueuse de la dispersion ou la phase aqueuse encapsulée des vésicules.

16. Composition selon la revendication 14 ou 15, caractérisée par le fait que les actifs lipophiles sont choisis parmi les sphingomyélines, les glycocéramides, et les céramides naturels ou synthétiques.

17. Composition selon l'une quelconque des revendications 1 à 16, caractérisée par le fait qu'elle contient une huile dispersée par les vésicules dans la phase aqueuse de la dispersion.

18. Composition selon la revendication 17, caractérisée par le fait que l'huile est choisie dans le groupe formé par:
- les huiles animales ou végétales ;
- les huiles essentielles naturelles ou synthétiques ;
- les hydrocarbures ;
- les carbures halogénés ;
- les silicones ;
- les esters d'acide minéral et d'un alcool ;
- les éthers et des polyéthers ;
- leurs mélanges.

19. Composition selon l'une quelconque des revendications 1 à 18, caractérisée par le fait qu'elle contient de 1 à 20 % en poids de pigment non-enrobé dispersé dans la phase aqueuse, par rapport au poids total de la composition.

20. Composition selon l'une quelconque des revendications 1 à 19, caractérisée par le fait la dispersion contient en plus des additifs choisis dans le groupe formé par les matifiants, des charges, des conservateurs, des gélifiants, des colorants, des actifs cosmétiques ou dermatologiques, hydrosolubles ou liposolubles.

21. Procédé de dispersion d'une charge organique et/ou d'une charge minérale pulvérulente dans une dispersion huile-dans-eau de vésicules lipidiques, caractérisé par le fait que l'on mélange la ou lesdites charges et la dispersion de vésicules au moyen d'un dispositif d'homogénéisation haute pression en un ou plusieurs passages.

22. Procédé selon la revendication 21, caractérisé par le fait que la pression utilisée varie de 4 à 8.10⁷ Pa et que la température d'utilisation est la température ambiante.

23. Procédé selon la revendication 21 ou 22, caractérisé par le fait que la charge est un pigment non-enrobé et que la dispersion de vésicules lipidiques est telle que définie dans l'une quelconque des revendications 1 à 20.

24. Procédé de préparation d'une composition selon l'une quelconque des revendications 1 à 20, caractérisé par le fait que l'on mélange à température ambiante une suspension aqueuse d'au moins un pigment non-enrobé à une dispersion huile-dans-eau de vésicules lipidiques à coeur aqueux dont la membrane est formée à partir d'au moins un ester d'acide gras par une homogénéisation classique suivie d'une homogénéisation selon le procédé défini dans la revendication 23.

25. Procédé selon la revendication 24, caractérisée par le fait que l'on refroidit après chaque passage dans l'homogénéisateur haute pression la dispersion obtenue par un courant d'air froid puis on désaère.

26. Composition cosmétique ou dermatologique, caractérisée par le fait qu'elle est constituée par la composition telle que définie selon l'une quelconque des revendications 1 à 20.

27. Utilisation d'une compositon selon l'une quelconque des revendications 1 à 20, comme base de produits pour le soin et/ou le maquillage du visage et/ou du corps et/ou du cuir chevelu.

28. Procédé de traitement cosmétique de la peau et/ou du cuir chevelu, caractérisée par le fait qu'on applique sur la peau ou le cuir chevelu une composition selon la revendication 26.

## Claims

1. Composition containing an aqueous dispersion of lipid vesicles with an aqueous core, characterized in that the dispersion comprises at least one uncoated pigment dispersed in the aqueous phase and that the lipid vesicles with an aqueous core have a membrane formed from at least one fatty acid ester.

2. Composition according to Claim 1, characterized in that it is an oil-in-water dispersion in which the lipid vesicles with an aqueous core act as an agent for dispersing droplets of oil in the dispersing aqueous phase.

3. Composition according to Claim 1 or 2, characterized in that the fatty acid esters constituting the membrane of the lipid vesicles with an aqueous core are chosen from the group formed by:
(i) polyol esters of fatty acid, which may or may not be polyoxyethylenated;
(ii) α-butylglycoside esters of fatty acid.

4. Composition according to any one of Claims 1 to 3, characterized in that the polyol esters of fatty acids are chosen from mixtures of at least one polyol chosen from the group formed by polyethylene glycol containing from 1 to 60 ethylene oxide units, sorbitan, sorbitan carrying 2 to 60 ethylene oxide units, glycerol carrying 2 to 30 ethylene oxide units, polyglycerols containing 2 to 15 glycerol units, sugars, glucoses carrying 2 to 30 ethylene oxide units, and at least one fatty acid containing a linear or branched, saturated or unsaturated C₅-C₁₇ alkyl chain, the number of alkyl chains per polyol group being between 1 and 10.

5. Composition according to Claim 3, characterized in that the α-butylglucoside esters of fatty acid contain a fatty chain of from 8 to 24 carbon atoms.

6. Composition according to Claim 5, characterized in that the α-butylglucoside esters of fatty acid are mixtures of α-butylglucoside esters of various fatty acids in which the various chains contain, relative to each other, a similar number of carbon atoms, or alternatively mixtures of α-butylglucoside mono-, di-, tri- or polyesters of the same fatty acid.

7. Composition according to Claim 5 or 6, characterized in that the α-butylglucoside ester of fatty acid is a mixture of α-butylglucoside mono- and diester of palmitic acid.

8. Composition according to any one of Claims 1 to 7, characterized in that the lipid vesicles with an aqueous core consist of a membrane formed from at least one fatty acid ester and from at least one ionic amphiphilic lipid.

9. Composition according to Claim 8, characterized in that the ionic amphiphilic lipids are chosen from neutralized anionic lipids, amphoteric lipids and alkylsulphonic derivatives.

10. Composition according to Claim 9, characterized in that the ionic amphiphilic lipids are chosen from the group formed by:
- alkaline salts of dicetyl phosphate and of dimyristyl phosphate;
- alkaline salts of cholesteryl sulphate;
- alkaline salts of lipoamino acids;
- the sodium salt of phosphatidic acid;
- phospholipids;
- alkylsulphonic derivatives of formula: 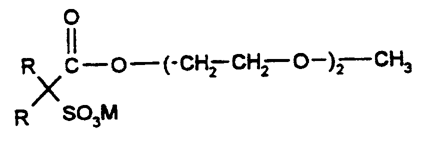 in which R represents C₁₂-C₂₂ radicals, in particular C₁₆H₃₃ and C₁₈H₃₇ radicals, taken as a mixture or separately, and M is an alkali metal, preferably sodium.

11. Composition according to any one of Claims 8 to 10, characterized in that the weight ratio between the amount of fatty acid ester and the amount of ionic amphiphilic lipid is between 50/1 and 50/25 and in that the weight ratio between the amount of lipid phase and the amount of aqueous phase is between 1/1000 and 300/1000.

12. Composition according to any one of Claims 1 to 11, characterized in that the lipid membrane of the vesicles with an aqueous core contains at least one additive.

13. Composition according to Claim 12, characterized in that the additive is chosen from the group formed by sterols, long-chain alcohols and diols, and long-chain amines and quaternary derivatives thereof.

14. Composition according to any one of Claims 1 to 13, characterized in that the lipid vesicles with an aqueous core contain at least one cosmetic and/or dermopharmaceutical active agent.

15. Composition according to Claim 13, characterized in that the lipid vesicles with an aqueous core encapsulate at least one water-soluble active agent and/or contain in the lipid membrane at least one lipophilic active agent and/or contain at least one amphiphilic active agent which is distributed between the membrane and the dispersing aqueous phase or the encapsulated aqueous phase of the vesicles.

16. Composition according to Claim 14 or 15, characterized in that the lipophilic active agents are chosen from sphingomyelins, glycoceramides and natural or synthetic ceramides.

17. Composition according to any one of Claims 1 to 16, characterized in that it contains an oil dispersed by vesicles in the dispersing aqueous phase.

18. Composition according to Claim 17, characterized in that the oil is chosen from the group formed by:
- animal or plant oils;
- natural or synthetic essential oils;
- hydrocarbons;
- halocarbons;
- silicones;
- inorganic acid esters of an alcohol;
- ethers and polyethers;
- mixtures thereof.

19. Composition according to any one of Claims 1 to 18, characterized in that it contains from 1 to 20 % by weight of uncoated pigment dispersed in the aqueous phase, relative to the total weight of the composition.

20. Composition according to any one of Claims 1 to 19, characterized in that the dispersion also contains additives chosen from the group formed by matt-effect agents, fillers, preserving agents, gelling agents, dyes and water-soluble or liposoluble cosmetic or dermatological active agents.

21. Process for dispersing an inorganic pulverulent filler and/or an organic filler in an oil-in-water dispersion of lipid vesicles, characterized in that the said filler or fillers and the vesicle dispersion are mixed using a high-pressure homogenizing device in one or more mixing runs.

22. Process according to Claim 21, characterized in that the pressure used ranges from 4 to 8 × 10⁷ Pa and that the temperature used is room temperature.

23. Process according to Claim 21 or 22, characterized in that the filler is an uncoated pigment and in that the dispersion of lipid vesicles is as defined in any one of Claims 1 to 20.

24. Process for the preparation of a composition according to any one of Claims 1 to 20, characterized in that an aqueous suspension of at least one uncoated pigment is mixed at room temperature with an oil-in-water dispersion of lipid vesicles with an aqueous core, in which the membrane is formed from at least one fatty acid ester, by a standard homogenization followed by a homogenization according to the process defined in Claim 23.

25. Process according to Claim 24, characterized in that after each passage through the high-pressure homogenizer, the dispersion obtained is cooled by a current of cold air and is then deaerated.

26. Cosmetic or dermatological composition, characterized in that it consists of the composition as defined according to any one of Claims 1 to 20.

27. Use of a composition according to any one of Claims 1 to 20, as a base for products to care for and/or make up the face and/or the body and/or the scalp.

28. Process for the cosmetic treatment of the skin and/or the scalp, characterized in that a composition according to Claim 26 is applied to the skin or the scalp.

## Patentansprüche

1. Zusammensetzungen, die eine wäßrige Dispersion von Lipidvesikeln mit innerem, wäßrigen Kompartiment enthalten,
dadurch gekennzeichnet, daß
die Dispersion mindestens ein nicht umhülltes, in der wäßrigen Phase dispergiertes Pigment enthält und dadurch, daß die Lipidvesikel mit innerem, wäßrigen Kompartiment eine Membran aufweisen, die aus mindestens einem Fettsäureester gebildet ist.

2. Zusammensetzungen nach Anspruch 1, dadurch gekennzeichnet, daß sie Öl-in-Wasser-Dispersionen sind, in der die Lipidvesikel mit innerem, wäßrigen Kompartiment als Dispergiermittel für Öltröpfchen in der wäßrigen Phase der Dispersion wirken.

3. Zusammensetzungen nach Anspruch 1 oder 2, dadurch gekennzeichnet, daß die Fettsäureester, die die Membran der Lipidvesikel mit innerem, wäßrigen Kompartiment bilden, ausgewählt sind unter:
(i) Polyol-Fettsäure-Estern, die gegebenenfalls poly ethoxyliert sind;
(ii) Fettsäureestern des α-Butylglucosids.

4. Zusammensetzungen nach einem der Ansprüche 1 bis 3, dadurch gekennzeichnet, daß die Polyol-Fettsäure-Ester unter den Gemischen mindestens eines Polyols, das unter Polyethylenglykol mit 1 bis 60 Ethylenoxideinheiten, Sorbitan, Sorbitan mit 2 bis 60 Ethylenoxideinheiten, Glycerin mit 2 bis 30 Ethylenoxideinheiten, Polyglycerin mit 2 bis 15 Glycerineinheiten, Zuckern und Glucosen mit 2 bis 30 Ethylenoxideinheiten ausgewählt ist, und mindestens einer Fettsäure, die eine gesättigte oder nicht gesättigte, geradkettige oder verzweigte C₅-₁₇-Alkylkette aufweist, ausgewählt sind; die Zahl der Alkylketten pro Polyolgruppe liegt im Bereich von 1 bis 10.

5. Zusammensetzungen nach Anspruch 3, dadurch gekennzeichnet, daß die Fettsäureester des α-Butylglucosids eine Fettkette mit 8 bis 24 Kohlenstoffatomen aufweisen.

6. Zusammensetzungen nach Anspruch 5, dadurch gekennzeichnet, daß die Fettsäureester des α-Butylglucosids Gemische von Estern verschiedener Fettsäuren mit α-Butylglucosid, deren unterschiedliche Ketten eine ähnliche Anzahl an Kohlenstoffatomen aufweisen, oder auch Gemische von Mono-, Di-, Tri- oder Polyestern einer einzigen Fettsäure mit α-Butylglucosid sind.

7. Zusammensetzungen nach Anspruch 5 oder 6, dadurch gekennzeichnet, daß der Fettsäureester des α-Butylglucosids ein Gemisch des Palmitinsäuremono- und -diesters des α-Butylglucosids ist.

8. Zusammensetzungen nach einem der Ansprüche 1 bis 7, dadurch gekennzeichnet, daß die Lipidvesikel mit innerem, wäßrigen Kompartiment aus einer Membran bestehen, die aus mindestens einem Fettsäureester und mindestens einem ionischen, amphiphilen Lipid gebildet wird.

9. Zusammensetzungen nach Anspruch 8, dadurch gekennzeichnet, daß die ionischen, amphiphilen Lipide ausgewählt sind unter den neutralisierten anionischen Lipiden, den amphoteren Lipiden oder den Alkylsulfonsäurederivaten.

10. Zusammensetzungen nach Anspruch 9, dadurch gekennzeichnet, daß die ionischen, amphiphilen Lipide ausgewählt sind unter:
- den Alkalisalzen von Dicetylphosphat und Dimyristylphosphat;
- den Alkalisalzen von Cholesterinsulfat;
- den Alkalisalzen von Lipoaminosäuren;
- dem Natriumsalz der Phosphatidsäure;
- den Phospholipiden;
- den Alkylsulfonsäurederivaten der folgenden Formel: 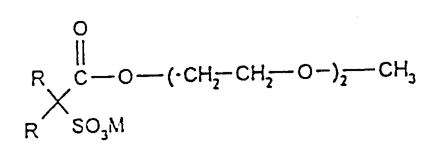 worin die Gruppen R gleichzeitig oder unabhängig voneinander Gruppen mit 12 bis 22 Kohlenstoffatomen bedeuten, insbesondere die Gruppen C₁₆H₃₃ und C₁₈H₃₇, und worin M ein Alkalimetall bedeutet, vorzugsweise Natrium.

11. Zusammensetzungen nach einem der Ansprüche 8 bis 10, dadurch gekennzeichnet, daß das Gewichtsverhältnis des Fettsäureesters zum ionischen, amphiphilen Lipid im Bereich von 50/1 bis 50/25 und das Gewichtsverhältnis der Lipidphase zur wäßrigen Phase im Bereich von 1/1000 bis 300/1000 liegt.

12. Zusammensetzungen nach einem der Ansprüche 1 bis 11, dadurch gekennzeichnet, daß die Lipidmembran der Vesikel mit innerem, wäßrigen Kompartiment mindestens einen Zusatzstoff enthält.

13. Zusammensetzungen nach Anspruch 12, dadurch gekennzeichnet, daß der Zusatzstoff unter den Sterinen, langkettigen Alkoholen und Diolen und den langkettigen Aminen und deren quaternären Derivaten ausgewählt ist.

14. Zusammensetzungen nach einem der Ansprüche 1 bis 13, dadurch gekennzeichnet, daß die Lipidvesikel mit innerem, wäßrigen Kompartiment mindestens einen kosmetischen und/oder dermopharmazeutischen Wirkstoff enthalten.

15. Zusammensetzungen nach Anspruch 14, dadurch gekennzeichnet, daß die Lipidvesikel mit innerem, wäßrigen Kompartiment mindestens einen wasserlöslichen Wirkstoff einkapseln und/oder in der Lipidmembran mindestens einen lipophilen Wirkstoff enthalten und/oder mindestens einen amphiphilen Wirkstoff enthalten, der in der Membran der wäßrigen Phase der Dispersion oder der von den Vesikeln eingekapselten wäßrigen Phase verteilt ist.

16. Zusammensetzungen nach Anspruch 14 oder 15, dadurch gekennzeichnet, daß die lipophilen Wirkstoffe unter den Sphingomyelinen, Glykoceramiden und den natürlichen oder synthetischen Ceramiden ausgewählt sind.

17. Zusammensetzungen nach einem der Ansprüche 1 bis 16, dadurch gekennzeichnet, daß sie ein Öl enthalten, das durch die Vesikel in der wäßrigen Phase der Dispersion dispergiert ist.

18. Zusammensetzungen nach Anspruch 17, dadurch gekennzeichnet, daß das Öl ausgewählt ist unter:
- den tierischen oder pflanzlichen Ölen;
- den natürlichen oder synthetischen etherischen Ölen;
- den Kohlenwasserstoffen;
- den halogenierten Kohlenstoffverbindungen;
- den Siliconen;
- den Estern einer anorganischen Säure und eines Alkohols;
- den Ethern und Polyethern
- oder deren Gemischen.

19. Zusammensetzungen nach einem der Ansprüche 1 bis 18, dadurch gekennzeichnet, daß sie, bezogen auf das Gesamtgewicht der Zusammensetzung, 1 bis 20 Gew.-% eines nicht umhüllten, in der wäßrigen Phase dispergierten Pigments enthalten.

20. Zusammensetzungen nach einem der Ansprüche 1 bis 19, dadurch gekennzeichnet, daß die Dispersion ferner Zusatzstoffe enthält, die unter den Mattierungsmitteln, Füllstoffen, Konservierungsmitteln, Gelbildnern, Färbemitteln und den kosmetischen oder dermatologischen, wasserlöslichen oder öllöslichen Wirkstoffen ausgewählt ist.

21. Verfahren zum Dispergieren eines pulverförmigen, anorganischen Füllstoffs und/oder eines organischen Füllstoffs in einer Öl-in-Wasser-Dispersion von Lipidvesikeln, dadurch gekennzeichnet, daß der Füllstoff oder die Füllstoffe und die Vesikeldispersion in einem oder in mehreren Durchgängen in einer Hochdruck-Homogenisierungsvorrichtung vermischt werden.

22. Verfahren nach Anspruch 21, dadurch gekennzeichnet, daß der angewandte Druck im Bereich von 4 bis 8 · 10⁷ Pa liegt und die Temperatur Raumtemperatur ist.

23. Verfahren nach Anspruch 21 oder 22, dadurch gekennzeichnet, daß der Füllstoff ein nicht umhülltes Pigment ist und dadurch, daß die Dispersion von Lipidvesikeln eine Dispersion nach einem der Ansprüche 1 bis 20 ist.

24. Verfahren zur Herstellung einer Zusammensetzung nach einem der Ansprüche 1 bis 20, dadurch gekennzeichnet, daß bei Raumtemperatur eine wäßrige Suspension mindestens eines nicht umhüllten Pigmentes mit einer Öl-in-Wasser-Dispersion von Lipidvesikeln mit innerem, wäßrigen Kompartiment, deren Membran aus mindestens einem Fettsäureester gebildet wird, durch herkömmliches Homogenisieren und anschließendes Homogenisieren nach dem Verfahren des Anspruchs 23 vermischt wird.

25. Verfahren nach Anspruch 24, dadurch gekennzeichnet, daß nach jedem Durchgang durch den Hochdruck-Homogenisator die erhaltene Dispersion mit einem kalten Luftstrom abgekühlt und anschließend entgast wird.

26. Kosmetische oder dermatologische Zusammensetzungen, dadurch gekennzeichnet, daß sie aus einer Zusammensetzung nach einem der Ansprüche 1 bis 20 gebildet werden.

27. Verwendung einer Zusammensetzung nach einem der Ansprüche 1 bis 20 als Basis für Produkte zur Pflege und/oder zum Schminken des Gesichts und/oder des Körpers und/oder der Kopfhaut.

28. Verfahren zur kosmetischen Behandlung der Haut und/oder der Kopfhaut, dadurch gekennzeichnet, daß auf die Haut oder die Kopfhaut eine Zusammensetzung nach Anspruch 26 aufgetragen wird.
